# EUROPEAN PATENT APPLICATION

(11) **EP 2 455 117 A1**
(43) Date of publication of application: **23.05.2012**
(21) Application number: 10191804.3
(22) Date of filing: 19.11.2010
(51) Int. Cl.: A61M 5/00, A61M 5/32

(54) **Assembly device for injection needles**

(71) Applicant: Sanofi-Aventis Deutschland GmbH, 65929 Frankfurt (DE)
(72) Inventor: The designation of the inventor has not yet been filed

(57) **Abstract**

The present invention relates to an assembly device for assembling and/or disassembling at least one needle unit (10) and a drug delivery device (30), wherein the assembly device comprises:
- a deposition means (26) adapted to bring or to keep the drug delivery device (30) in an assembly position,
- a holder (44) for at least one needle unit (10), and
- an assembly unit (38) adapted to assemble and/or to disassemble the needle unit (10) and the drug delivery device (30).

## Description

### Field of the Invention

The present invention relates to drug delivery devices and in particular to pen-type injectors, wherein a removable piercing or needle assembly is to be manually attached to an outlet end of the drug delivery device.

### Background and Prior Art

User operated drug delivery devices are typically applicable in circumstances, where persons without formal medical training, in particular patients, need to administer an accurate and predefined dose of a medicament, such as heparin or insulin. Such devices are typically applied, where the medicament is to be administered on a regular or irregular basis during a short or long-term period.

Generally, such devices should be robust in construction, jet easy to use in terms of handling and in understanding by the user of its operation and the delivery of the required dose. Furthermore, the dose setting must be easy and unambiguous. When the device is to be disposable rather than reusable, it should be inexpensive to manufacture and easy to dispose. To meet these requirements, the overall number of parts required to assemble the device should be kept on a minimum.

The injectable medicament to be dispensed by way of such drug delivery devices is typically provided in a disposable or replaceable cartridge, such as a vial, ampoule or carpule comprising a barrel, typically of cylindrical geometry, and a piston slidably disposed therein for sealing the cartridge.

Drug delivery devices, such like pen-type injectors typically comprise a housing having a cartridge holder for receiving the cartridge at least partially filled with the medicinal product which is intended to be dispensed. The distal end section of a cartridge holder facisng towards the patient during an injection procedure typically comprises a through opening that provides access to a sealed distal end of the cartridge. By way of said through opening, an injection needle or cannula may penetrate the elastic seal of the cartridge to establish a fluid interconnect allowing the medicinal product to be expelled from the cartridge.

Typically, the disposable injection needle is provided by way of a needle assembly for releasably fastening the injection needle to the cartridge holder. The needle assembly typically comprises a needle hub supporting the injection needle and having further a cupped receptacle with an inner thread to be screwed onto a correspondingly threaded distal socket of the cartridge holder. The needle hub is typically provided with a removable needle cap serving as a protection means for preventing inadvertent stitch damage.

For administering a dose of the medicament, the needle cap has to be removed, before the needle penetrates the skin of the patient. Upon completion of the medicament injection procedure, the needle is removed from the biological tissue and prior to disassemble needle hub and cartridge holder, an outer needle cap should be mounted on the needle hub for the purpose of protecting the patient or the user. Inadvertent stitch damages may particularly occur, when the patient mounts the needle cap back on the needle hub. This problem is getting even more severe when the respective patient is physically infirm, has impaired vision and/or suffers other side effects.

### Objects of the Invention

It is therefore an object of the present invention to facilitate and to simplify the assembly procedure, in particular for screwing or unscrewing a needle holder or needle unit to or from a cartridge holder of a drug delivery device. In particular, stitch damages arising during assembly or disassembly of a needle unit should be entirely prevented. Moreover, the general device handling of pen-type injectors should be simplified and an assembly or disassembly procedure for a needle unit should be conducted with high precision.

### Summary of the Invention

In a first aspect, the invention provides an assembly device for assembling and/or disassembling at least one needle unit and a drug delivery device. The assembly device comprises a deposition means being adapted to bring or to keep the drug delivery device in a pre-defined assembly position with respect to an assembly unit. The assembly device further has a holder for at least one needle unit. The holder typically has at least one receptacle adapted to receive the needle unit to be assembled with the drug delivery device.

The assembly device further comprises an assembly unit which is adapted to assemble and/or to disassemble the needle unit and the drug delivery device. Preferably, deposition means, holder and assembly unit interact in an autonomous or automatic way, such, that by simply arranging the drug delivery device in the assembly position, a needle unit can be automatically assembled to or disassembled from the drug delivery device, without any further interaction of the user.

The deposition means may define a particular spot or position on or in the assembly device, where the drug delivery is accessible for the assembly unit in order to assemble or to disassemble drug delivery device and needle unit.

In a more sophisticated embodiment, the deposition means may fetch the drug delivery device and may autonomously arrange the drug delivery device into the required assembly position.

The assembly device is generally adapted to assemble and/or to disassemble the drug delivery device and the interchangeable needle unit. Since the process of assembly and/or disassembly is entirely conducted by the assembly device, the likelihood or danger of stitch damages can be remarkably reduced because manual handling of the needle unit is no longer required in the process of assembly or disassembly.

In a preferred embodiment, the assembly device further comprises a needle preparation unit which is adapted to remove a protecting foil from the needle unit prior to an assembly of drug delivery device and needle unit. By means of the needle preparation unit, commercially distributed needle units protected with inner and/or outer needle caps and/or provided with a protecting foil typically covering a proximal portion of a needle hub to be screwed on the drug delivery device can be inserted into the holder by a user without any further requirements regarding needle preparation. Removal of a protecting foil and/or of inner and/or outer needle cap can be entirely provided by the assembly device.

In this way, the end user does not get in direct contact with non-protected needle tips, and the likelihood or danger of stitch damages to occur can be further reduced.

According to another preferred embodiment, the holder is moveably disposed in a housing of the assembly device for transporting the at least one needle unit towards and/or away from the assembly unit. The holder itself acts as a kind of feeder and by moving the same in a feeding direction unused and new needle units can be transported towards the assembly unit in a similar way as used needle units can be returned to the holder for transporting them away from the assembly unit. Handling of both, new and unused as well as used needles and their respective transport inside the assembly device can be provided with a single holder, preferably comprising numerous receptacles, each of which being adapted to receive a needle unit.

In another embodiment, the needle preparation unit and the assembly unit are disposed on opposite sides of the holder. For instance, the needle preparation unit is arranged on that side of the holder, from which the protecting foils covering a needle hub are accessible, whereas the assembly unit is disposed on the opposite side of the holder, e.g. in order to grip a distal end of the needle unit and to screw the needle unit onto a distally located outlet end of the drug delivery device.

In this context, the terms "distal" and "proximal" relate to the geometry of the drug delivery device. In operation of the device, the "distal" direction points towards the skin of a patient whereas the "proximal" direction points in an opposite direction, hence away from a treatment area of the patient's skin.

According to another preferred embodiment, the assembly unit is further adapted to take a needle unit from the holder and to screw the needle unit onto a distal end section of the drug delivery device

Alternatively or additionally, the assembly unit is further adapted to engage with a needle unit assembled with the drug delivery for the purpose of disassembling needle unit and delivery device and to place the disassembled needle unit into a receptacle or slot of the holder. The assembly device preferably comprises a detection means in order to detect, whether the drug delivery device is already equipped with a needle unit or not.

If the drug delivery device is positioned in the assembly position and if it is detected, that a used needle is already assembled thereto, the assembly unit will be switched into a disassembling mode in order to disassemble needle unit and drug delivery device. Otherwise, if the drug delivery device is put in assembly position without a needle unit assembled thereto, the assembly device will be automatically switched into an assembling mode and the assembly unit will autonomously take a needle unit from the holder and will assemble the needle unit to the drug delivery device.

In a further preferred embodiment, the assembly unit is arranged in a longitudinal and imaginary extension of the drug delivery device's longitudinal axis, when the drug delivery device is disposed in assembly position with respect to the assembly device. Preferably, the holder providing the needle units is movably disposed between the drug delivery device and the assembly unit.

In still another embodiment, the assembly unit and/or the needle preparation unit are autonomously activated in response to place the drug delivery device in assembly position. Hence, the assembly device comprises at least one detector or sensor adapted to detect when the drug delivery device reaches the pre-defined assembly position.

In still another embodiment, the holder comprises several slots or receptacles, each of which being adapted to receive a needle unit.

The slots or receptacles are arranged on the holder in such a manner that corresponds to a feeding direction of the holder. This means, adjacent or neighbouring slots are geometrically arranged in a way that corresponds to the feeding direction of the holder. If for instance the holder is to be displaced in a translational or longitudinal way through the housing of the assembly device, the respective slots or receptacles of the holder are arranged along an imaginary straight line parallel to the feeding direction.

Otherwise, if for instance the holder is rotationally mounted in the assembly device, the various slots or receptacles for the needle units are preferably disposed in a circumferential way. Accordingly, the assembly unit is rotationally and/or slidably mounted with respect to the longitudinal axis of the drug delivery device. In case of a rotational support, rotation axis of the assembly unit is substantially parallel to the longitudinal axis of the drug delivery device.

Irrespective on whether the holder is translationally or rotationally supported in or on the assembly device, the feeding direction of the holder is preferably arranged substantially perpendicular to the direction along which the assembly unit moves for assembling and/or disassembling needle unit and drug delivery device. Instead of a substantially perpendicular arrangement, it is also conceivable, that the general feeding direction and the direction of displacement of the assembly unit extend at an angle with respect to each other.

According to another preferred aspect, the holder comprises a rather straight shaped bar-like body having numerous needle unit receiving receptacles or slots disposed therein.

The bar-like body may be rather flat in shape, such that the needle unit with its distal end section protrudes from said body and thus enables an easy gripping of a needle unit by way of the assembly unit.

Furthermore, and according to another preferred aspect, the holder is frictionally and/or positively engaged with a drive means for successively displacing the holder in feeding direction. The drive means is preferably coupled with the assembly unit and provides a step-wise feeding or movement of the holder and the needle units disposed thereon.

In still another embodiment, the housing of the assembly device comprises an inserting opening and an ejecting opening arranged on opposite sides of the housing for receiving and/or ejecting the holder, respectively.

This way, the housing of the assembly device provides a passage for the holder, to be equipped with a set of new and unused needle units. The loaded holder can be placed into the receiving or inserting opening and may after usage step-wise ejected from the oppositely located ejecting opening. The assembly device and the holder can be designed such that the holder at least partially protrudes from the housing of the assembly device when the very first and/or when very last needle units arranged at opposite end sections of the holder are assembled or disassembled by the assembly device, respectively.

In still another but independent aspect the invention further relates to a holder for a number of needle units comprising a respective number of needle unit receiving slots or receptacles, wherein said holder is adapted to be coupled with or is to be inserted into an above described assembly device.

It will be further apparent to those skilled in the pertinent art that various modifications and variations can be made to the present invention without departing from the spirit and scope of the invention. Further, it is to be noted, that any reference signs used in the appended claims are not to be construed as limiting the scope of the present invention.

### Brief Description of the Drawings

In the following, preferred embodiments of the invention will be described in greater detail by making reference to the drawings in which:
- Figure 1: schematically illustrates a needle unit of a drug delivery device,
- Figure 2: schematically shows the assembly device for automatically assembling and/or disassembling a drug delivery device with a needle unit according to Figure 1, and
- Figure 3: schematically shows a holder adapted to receive numerous needle units according to Figure 1.

### Detailed Description

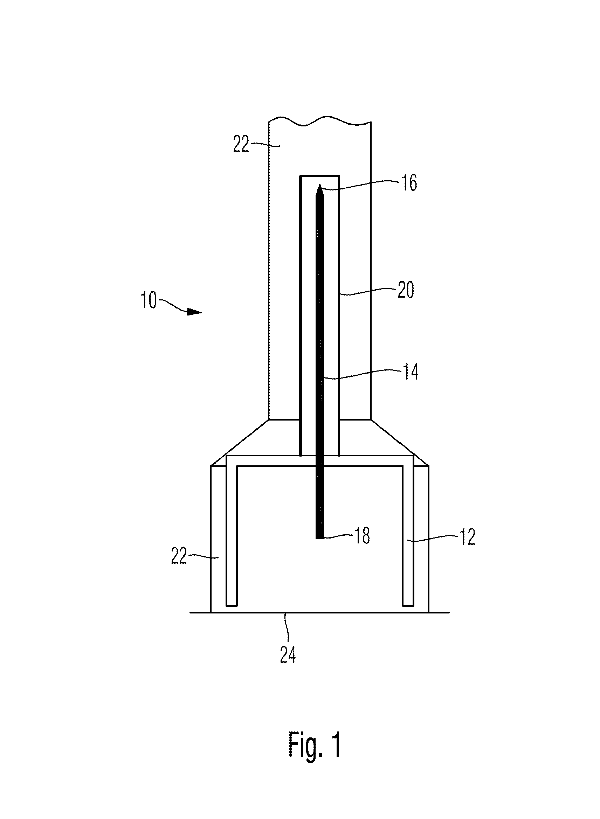

In Figure 1, a typical embodiment of a needle unit 10 is illustrated. The needle unit comprises a piercing element 14 e.g. in form of an injection needle or cannula. The needle 14 with its distal end 16 is intended to penetrate biological or dermal tissue, whereas the proximal end 18 of the needle 14, which may be also tipped, is intended to penetrate a seal of a cartridge containing a medicament to be dispensed by the drug delivery device. The needle 14 is mounted in a needle hub 12 having for instance a threaded section inside its annular side wall. By way of said threaded section, the needle hub 12 can for instance be screwed on a corresponding needle holder 34 of a drug delivery device.

When commercially distributed, the needle unit 10 is provided with a protective foil 24 covering the inner volume of the needle hub 12 and thus preventing a stitching at the proximal tipped end 18 of the needle 14. Additionally, the entire needle unit 10 is protected by an outer needle cap 22 covering the elongated needle section 14 as well as the radially widened needle hub 12. When the outer needle cap 22 is removed, another, inner needle cap 20 having a shaft-like geometry is still disposed around the tipped needle 14. Prior to an injection, this inner needle cap 20 has to be removed. While the inner needle cap is intended to be discarded, the outer needle cap 22 can or even should be put back onto the needle hub 12 in order to avoid stitch damage after usage and/or during disassembling of the needle unit and drug delivery device 30.

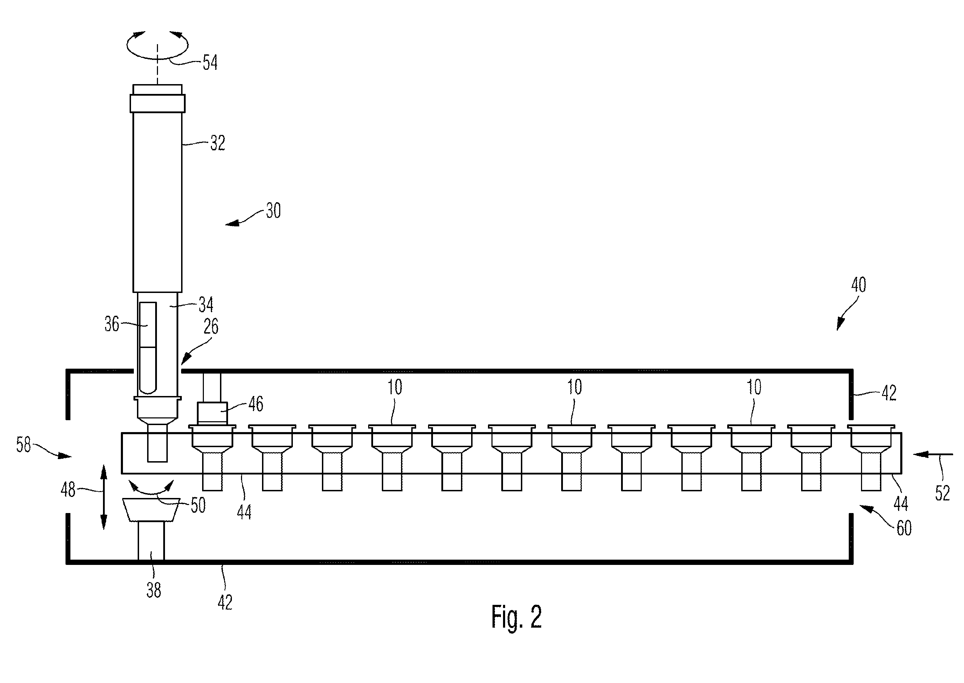

In Figure 2, the assembly device 40 for automatically assembling and/or disassembling drug delivery device 30 and needle unit 10 is shown. The assembly device 40 has a housing 42 comprising an inserting opening 60 and an ejecting opening 58 arranged at opposite sides of the housing 42. Said openings 58, 60 form a passage that allows to subsequently feed of push a bar-like holder 44 equipped with numerous needle units 10 through the housing 42 of the assembly device 40.

At a left-handed section of the assembly device 40 as illustrated in Figure 2, there is provided an assembly unit 38, which is adapted to rotate along the long axis of the drug delivery device 30 as illustrated by the bended arrow 50. Additionally, the assembly unit 38 is free to move in vertical direction 48 in order to screw the needle unit 10 to a distal and downward pointing needle holder section 34 of the drug delivery device 30. Additionally or alternatively, as indicated in Figure 2 it is also conceivable, to rotate the drug delivery device 30 with respect to its longitudinal axis as indicated by the bended arrow 54.

Since the assembly and disassembly of needle unit 10 and cartridge holder 34 takes place inside the housing 42 of the assembly device 40, a potential danger of stitching can be advantageously reduced. Handling of the assembly device only requires to insert the drug delivery device 30, at least its distally located cartridge holder 34, into a through opening 26 disposed in an upper section of the housing 42. In the illustrated embodiment said through opening 26 forms a deposition means, which is adapted to keep the drug delivery device 30 in a pre-defined assembly position in which assembly or disassembly of the needle unit 10 can be conducted.

The illustrated assembly device 40 is particularly designed for a drug delivery device, such like a pen-type injector 30 having a proximal housing section 32, a distal cartridge holder section 34 with an inspection window 36 allowing to visually inspect the filling level of a cartridge disposed in the cartridge holder 34. However, the assembly device may also be generally adapted and used with other types of drug delivery devices that require removable assembly of a needle unit 10.

As further illustrated in Figure 2, the assembly device 40 also comprises a preparation unit 46 arranged at a lateral distance with respect to the through opening or aperture 26. Since the holder 44 is to be displaced stepwise in the feeding direction 52, hence from the right to the left in Figure 2, it is of particular benefit, that the preparation unit 46, being at least intended to remove the protecting foil 24 from the needle hub 12, is arranged in direct vicinity to the assembly unit 38. This way, the protecting foil 24, which also prevents contamination of the needle unit 10 is to be removed from the needle hub 12 just prior to an assembly with the cartridge holder 34 of the drug delivery device 30.

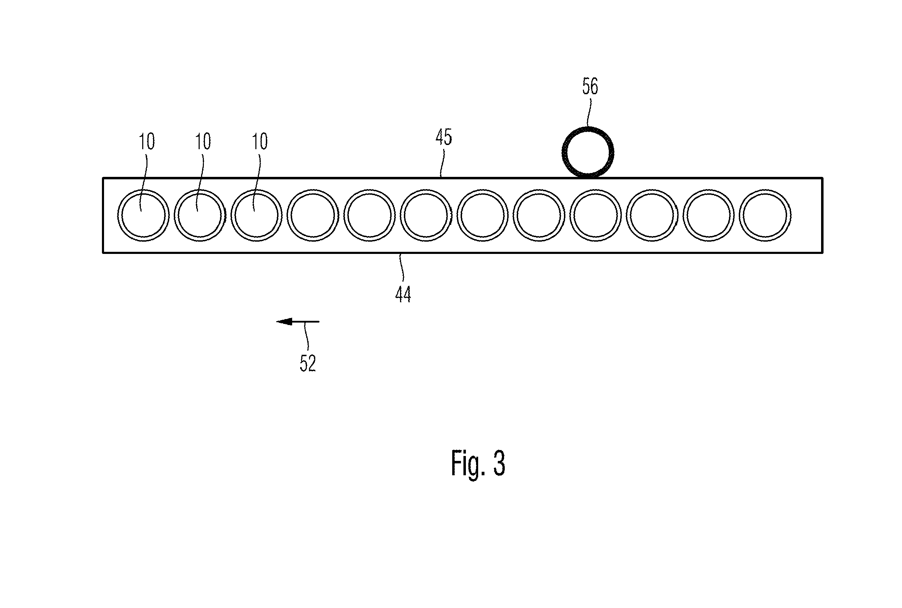

The feeding or transport of the holder 44 through the assembly device 40 is exemplary illustrated in the sketch of Figure 3, where the bar-like holder 44 is depicted from above. Here, a linear, translational displacement of the holder 44 is attained by means of a drive member 56 frictionally and/or positively engaged with a side section 45 of the holder 44. Said side face 45 may be toothed and the drive means may comprise a respectively toothed gear. Alternatively, the side 45 may frictionally engage with the drive member 56 and may therefore comprise a suitable surface finish at its lateral side section.

Moreover but not explicitly illustrated in the Figures, the assembly device 40 comprises a control unit adapted to control the feeding of the holder 44 as well as the screwing and/or unscrewing procedure conducted by the assembly unit 38. Preferably, rotation angle as well as momentum for screwing and/or unscrewing the needle unit 10 onto or from the cartridge holder 34 is monitored in order to provide a precise and secure fastening and assembly of needle unit 10 and cartridge holder 34.

Even though the assembly device 40 is exemplary explained and illustrated by means of a screwing assembly and disassembly, also other ways and methods of mutually assembling the needle unit 10 and the drug delivery device 30 are generally conceivable and may be conducted by the assembly device 40.

### List of Reference Numerals

- 10: needle assembly
- 12: needle hub
- 14: injection needle
- 16: distal tip
- 18: proximal tip
- 20: inner needle cap
- 22: outer needle cap
- 24: protecting foil
- 26: through opening
- 30: drug delivery device
- 32: housing
- 34: cartridge holder
- 36: inspection window
- 38: assembly unit
- 40: assembly device
- 42: housing
- 44: holder
- 45: side surface
- 46: preparation unit
- 48: displacement direction
- 50: screwing direction
- 52: feeding direction
- 54: screwing direction
- 56: drive member
- 58: ejecting opening
- 60: inserting opening

## Claims

1. An assembly device for assembling and/or disassembling at least one needle unit (10) and a drug delivery device (30), wherein the assembly device comprises:
- a deposition means (26) adapted to bring or to keep the drug delivery device (30) in an assembly position,
- a holder (44) for at least one needle unit (10), and
- an assembly unit (38) adapted to assemble and/or to disassemble the needle unit (10) and the drug delivery device (30).

2. The assembly device according to claim 1, further comprising a needle preparation unit (46), adapted to remove a protecting foil (24) from the needle unit (10) prior to an assembly of drug delivery device (10) and needle unit (30).

3. The assembly device according to any one of the preceding claims, wherein the holder (44) is moveably disposed in a housing (42) of the assembly device for transporting the at least one needle unit (10) towards and/or away from the assembly unit (38).

4. The assembly device according to any one of the preceding claims 2 or 3,
wherein the needle preparation unit (46) and the assembly unit (38) are disposed on opposite sides of the holder (44).

5. The assembly device according to any one of the preceding claims, wherein the assembly unit (38) is adapted to take a needle unit (10) from the holder (44) and to screw the needle unit (10) on a distal end section (34) of the drug delivery device (30).

6. The assembly device according to any one of the preceding claims, wherein the assembly unit (38) is arranged in a longitudinal extension of the drug delivery device when disposed in assembly position.

7. The assembly device according to any one of the preceding claims, wherein the assembly unit (38) and/or the needle preparation unit (46) are autonomously activated in response to place the drug delivery device (30) in assembly position.

8. The assembly device according to any one of the preceding claims, wherein the holder (44) comprises several slots adapted to receive a needle unit (10).

9. The assembly device according to claim 8, wherein the slots are arranged in a manner that corresponds to a feeding direction (52) of the holder (44).

10. The assembly device according to any one of the preceding claims, wherein the assembly unit (38) is rotationally and/or slidably mounted with respect to the longitudinal axis of the drug delivery device (30) if disposed in assembly position.

11. The assembly device according to any one of the preceding claims, wherein the assembly unit (38) is movable disposed in a direction substantially perpendicular to the feeding direction (52) of the holder (44).

12. The assembly device according to any one of the preceding claims, wherein the holder (44) comprises a straight shaped bar-like body having numerous needle unit (10) receiving slots disposed therein.

13. The assembly device according to any one of the preceding claims, wherein the holder (44) is frictionally and/or positively engaged with a drive means (56) for displacing the holder (44) in feeding direction (52).

14. The assembly device according to any one of the preceding claims 3 to 13, wherein the housing (42) comprises an inserting opening (60) and an ejecting opening (58) arranged on opposite sides of the housing (42) for receiving and/or ejecting the holder (44).

15. A holder for a number of needle units (10) comprising a respective number of needle unit (10) receiving slots and being further adapted to be inserted into an assembly device (40) according to any one of the preceding claims.
